Europäisches Patentamt

European Patent Office

Office européen des brevets

(19)

(11) Veröffentlichungsnummer : **0 073 079**
**B1**

(12) # EUROPÄISCHE PATENTSCHRIFT

(45) Veröffentlichungstag der Patentschrift :
26.03.86

(21) Anmeldenummer : 82200996.5

(22) Anmeldetag : 09.08.82

(51) Int. Cl.⁴ : **C 12 M 1/12**

(54) **Verfahren und Vorrichtung zur Züchtung von Mikroorganismen.**

(30) Priorität : 18.08.81 CH 5327/81

(43) Veröffentlichungstag der Anmeldung :
02.03.83 Patentblatt 83/09

(45) Bekanntmachung des Hinweises auf die Patenterteilung : 26.03.86 Patentblatt 86/13

(84) Benannte Vertragsstaaten :
**AT CH DE FR GB IT LI NL SE**

(56) Entgegenhaltungen :
**CH-A- 563 457
DE-A- 2 140 159
DE-B- 1 156 051
FR-A- 2 352 055
FR-A- 2 450 567
NL-A- 7 903 589
US-A- 2 710 099
US-A- 3 926 737
US-A- 4 184 916
CHEMIE-ING.-TECHN., Band 36, Nr. 12, Dezember 1964, Seiten 1285-1292 S. WERNER et al.: "Filterapparate"
Houben-Weyl, Band I (1958), Seite 165**

(73) Patentinhaber : **DrM, Dr. Müller AG
Alte Landstrasse 421
CH-8708 Männedorf ZH (CH)**

(72) Erfinder : **Müller, Hans, Dr.
Im Allmendli
CH-8703 Erlenbach .(CH)**

(74) Vertreter : **Herrmann, Peter Johannes
Bauernhalde 9
CH-8708 Männedorf ZH (CH)**

## Beschreibung

Die Erfindung betrifft ein Verfahren und eine Vorrichtung zur Züchtung von Mikroorganismen in einem Fermenter, wobei das verbrauchte Substrat kontinuierlich abfiltriert wird.

Bei den zu züchtenden Mikroorganismen handelt es sich um Hefen, Bakterien oder Pilze, welche in einem wässerigen Substrat aufgeschlämmt, vermehrt werden. Insbesondere bei kontinuierlicher Züchtung kann das Substrat, beispielsweise eine Zuckerlösung, mit den notwendigen Nährstoffen in relativ verdünnter Form zugegeben werden. Vielfach fallen die Nährlösungen in sehr verdünnter Form an. Anderseits darf eine gewisse Konzentration an Stoffwechselprodukten nicht überschritten werden, da sonst die Mikroorganismen vergiftet und demzufolge das Wachstum gehemmt oder ganz unterbunden wird.

Nach der CH-PS 621 363 ist ein Verfahren bekannt, bei welchem zur Erhöhung der Zellkonzentration im Fermenter die Substratflüssigkeit mit Hilfe eines Anschwemmfilters abgetrennt und die Mikroorganismen nach Abtrennung der Substratflüssigkeit zusammen mit dem Filterhilfsmittel in den Fermenter zurückgeführt werden. Fermentierung und Abtrennung sowie Rückführung erfolgen in getrennten Apparaten mit externen Rohrleitungen und müssen unter sterilen Bedingungen durchgeführt werden.

Die Durchführung von sterilen Fermentierungen wirft in Fermentern zunehmender Grösse an sich schon Probleme auf. In einem Zuchtgerät sind zahlreiche Sicherheitsmassnahmen zu beachten. Daher erhöht ein Verfahren mit externer Filtration und den erforderlichen Rohrleitungen und Ventilen zur Verbindung beider Apparate die Kontaminationsgefahr der zu züchtenden Mikroorganismen mit Fremdkeimen in erheblichem Ausmass.

Aufgabe der Erfindung ist es, ein Verfahren zur Züchtung von Mikroorganismen unter gleichzeitiger Konzentrierung derselben innerhalb eines Züchtungsbehälters zu schaffen, sowie einen Fermenter zur Durchführung des Verfahrens.

Diese Aufgabe wird erfindungsgemäss nach Anspruch 1 gelöst und ist dadurch gekennzeichnet, dass die Mikroorganismen über im Innern des Fermenters angeordnete Anschwemmkerzenfilterelemente abfiltriert und die abgelagerten Mikroorganismen durch Rückspülung von den Geweben der Anschwemmkerzenfilterelemente abgeworfen werden.

Auf diese Weise kann die Konzentration an Stoffwechselprodukten während der Fermentierung durch andauernde Entfernung des Substrates erniedrigt und anderseits die Mikroorganismen ebenfalls kontinuierlich entfernt werden. Es kann so bei viel höherer Konzentration an Mikroorganismen gearbeitet werden, was eine Vervielfachung der Produktivität entspricht.

Die von den Anschwemmkerzenfilterelementen abgeworfenen eingedickten Mikoorganismen sammeln sich im konischen Teil des Fermenters und können von dort kontinuierlich oder chargenweise abgezogen werden. Dadurch, dass die Filterelemente gruppenweise abschaltbar sind, können bei der Rückspülung einzelner Gruppen, die übrigen Filterelemente weiter filtrieren. Dabei kann im Fermenter Ueberdruck, Normaldruck oder Unterdruck herrschen. Auch ist es möglich, während der Fermentierung ein biologisch inertes Filterhilfsmittel zu dosieren. Die Anlage kann mittels Computer vollautomatisch gesteuert werden.

Anordnung und Anzahl der Filterelemente hängt weitgehend von der Grösse des Fermenters ab. Es können mehrere, gruppenweise verbundene Filterelemente vorgesehen sein, welche auch gruppenweise Filtratabflüsse und Rückspülanschlüsse aufweisen, welche einzeln betätigt werden können. Sie können aber auch ringförmig um ein belüftetes Zentralrohr oder Rührelement angeordnet sein. Die Filterelemente können als Kerzen ausgebildet sein, die mit Gewebe überzogen sind. Oberhalb der Filterelemente ist zur Regulierung der Druckverhältnisse ein Gasraum vorgesehen.

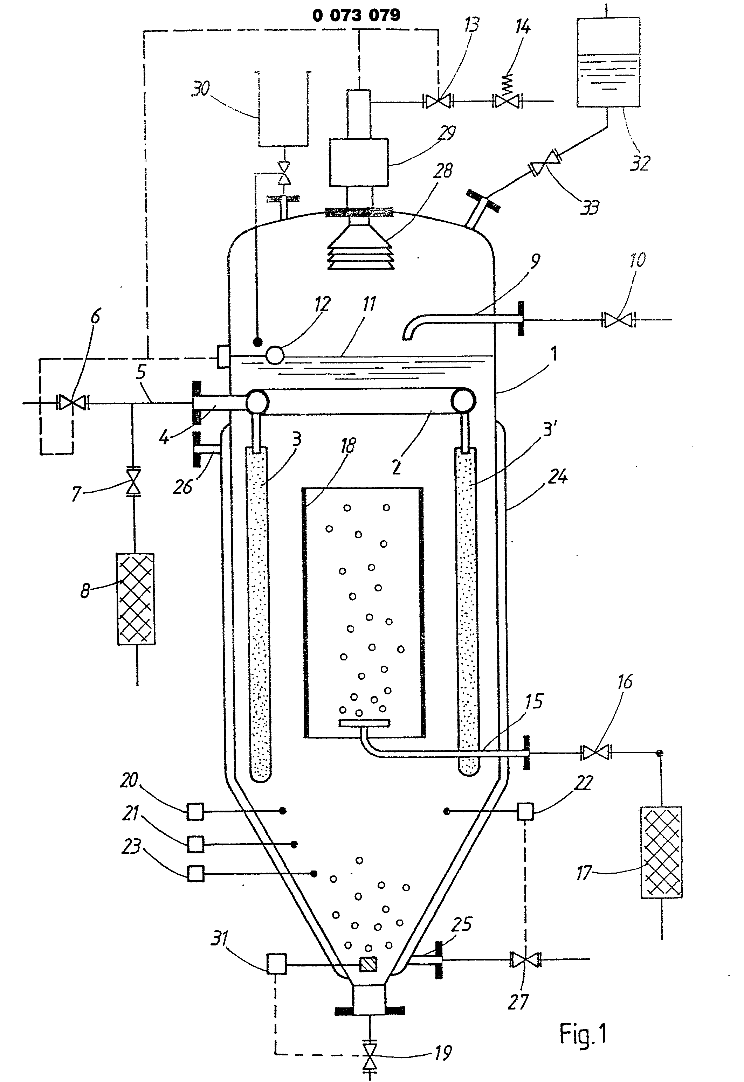

Die Arbeitsweise soll anhand der einzigen Figur erläutert werden:

In einem Fermenterbehälter 1 sind an einem Ringrohr 2 Anschwemmkerzenfilterelemente 3, 3', befestigt. Sie haben entweder einen gemeinsamen Anschluss 4 nach aussen oder sind in Gruppen aufgeteilt, wobei jeder Gruppe ein solcher Anschluss zukommt. Ausserhalb des Fermenterbehälters 1 mündet das Ringrohr 2 in ein Sammelrohr 5, welches mit zwei Ventilen versehen ist. Es sind dies ein Austrittsventil 6, aus welchem das fermentierte Substrat austritt und ein Luft- oder Flüssigkeitsventil 7 für die Rückspülung der Filterelemente 3. Das Rückspülmedium kann bei steriler Arbeitsweise mittels eines Filters 8 sterilisiert werden. Der Zulauf des Substrates (Kohlenstoffquelle und Nährsalze), wird durch die Rohrleitung 9 über das Eintrittsventil 10 geregelt. Das Flüssigkeitsniveau 11 im Fermenterbehälter 1 wird durch einen Regler 12 durch Betätigung des Abluftventils 13, welchem ein Druckhalteventil 14 nachgeschaltet ist, einerseits und das Austrittsventil 6 anderseits konstant gehalten.

Sofern es sich um Züchtung von aeroben Mikroorganismen handelt, wird die Luft durch eine Begasungsvorrichtung 15 und das Ventil 16 über ein Filter 17 zugegeben. Diese Luft wirkt gleichzeitig als Umwälzmedium in dem zentralen Umwälzrohr 18. Sofern es sich um grosse Apparate handelt und eine genügend grosse Anzahl von Filterelementen 3 eingebaut werden können, reicht oftmals die Rückspülluft zur Belüftung aus.

Im Konus des Fermenters 1 befindet sich ein Ablassventil 19, mit Hilfe dessen die Mikroorganismen abgezogen werden können. Das Ventil 19 wird in Sequenz gesteuert, wobei diese Sequenz massgebend ist für die Konzentration der Mikroorganismen. Der Fermenter 1 ist zudem mit den in der mikrobiologischen Züchtung üblichen Instrumenten, beispielsweise einer pH-sonde 20, einer Sauerstoffelektrode 21, einem Kontaktthermometer 22, einer Redoxelektrode 23 versehen.

Zur Abführung der Reaktionswärme besitzt der Fermenter 1 einen Kühlmantel 24 mit einem Stutzen 25 für den Kühlwassereintritt und einen Stutzen 26 für den Kühlwasseraustritt. Die Kühlwassermenge wird über die Temperatursonde 22 und ein Ventil 27 geregelt. Bei grösseren Fermentern sind zusätzlich interne Kühlelemente eingebaut.

Die Filterelemente 3 sind mit Textil- oder Kunststoffgewebe überzogen, welche Oeffnungen von 1-30 μm aufweisen.

Für Bakterien können auch Filterkerzen mit feineren Membranen, beispielsweise Teflon® (Warenzeichen von Du Pont) eingesetzt werden, wie diese in der Sterilfiltrationstechnik Verwendung finden. Im Kopf des Fermenters 1 wird die Abluft durch einen mechanischen Zentrifugal-Schaumabscheider 28 geführt, welcher durch einen Motor 29 angetrieben wird. Zusätzlich kann eine automatische Zudosierung von Antischaummitteln aus einem Behälter 30 vorgesehen werden. Die Konzentration der austretenden Mikroorganismen kann durch ein Instrument 31 gemessen werden, wobei dieser Messwert die Oeffnung des Ablassventiles 19 steuert. Die Impfung des Apparates erfolgt aus einem Gefäss 32 über ein Ventil 33.

Die Produktivität wird wegen der hohen Zellkonzentration auf ein Mehrfaches gesteigert.

Bei bekannten Fermentern kann aus einer 4 %-igen Zuckerlösung eine Aufschlämmung von 2 % Hefe erreicht werden. Beim erfindungsgemässen Verfahren werden Hefekonzentrationen von 10-15 % erreicht.

Anstelle eines Ueberdruckes kann am Ventil 6 auch Vakuum angesetzt werden.

## Patentansprüche

1. Verfahren zur kontinuierlichen Züchtung von Mikroorganismen in einem Fermenter, wobei das verbrauchte Substrat kontinuierlich abfiltriert wird, dadurch gekennzeichnet, dass die Mikroorganismen über im Inneren des Fermenters (1) angeordnete Anschwemmkerzenfilterelemente (3, 3') abfiltriert und die abgelagerten Mikroorganismen durch Rückspülung von den Geweben der Anschwemmkerzenfilterelemente (3, 3') abgeworfen werden.

2. Verfahren nach Anspruch 1, dadurch gekennzeichnet, dass die abgeworfenen eingedickten Mikroorganismen sich im unteren Teil des Fermenters ansammeln und von dort kontinuierlich oder diskontinuierlich abgezogen werden.

3. Verfahren nach Ansprüchen 1 und 2, dadurch gekennzeichnet, dass die Anschwemmkerzenfilterelemente 3 gruppenweise verbunden sind und die Rückspülung so erfolgt, dass einzelne Gruppen immer in Filtration bleiben.

4. Verfahren nach den Ansprüchen 1 bis 3, dadurch gekennzeichnet, dass für die Filtration im Fermenter mit Ueberdruck gearbeitet wird.

5. Verfahren nach den Ansprüchen 1 bis 3, dadurch gekennzeichnet, dass im Fermenter bei Normaldruck gearbeitet wird und die Filtration durch Unterdruck in der Filterleitung erfolgt.

6. Vorrichtung zur Durchführung des Verfahrens nach Anspruch 1, dadurch gekennzeichnet, dass in einem Fermentierbehälter (1) mehrere, gruppenweise verbundene Anschwemm-Kerzenfilterelemente (3) vorgesehen sind, welche mit Gewebenüberzogen sind und gruppenweise Filtratabflüsse und Rückspülanschlüsse aufweisen, und welche einzeln betätigt werden können.

7. Vorrichtung nach Anspruch 6, dadurch gekennzeichnet, dass die Anschwemm-Kerzenfilterelemente (3) ringförmig um ein belüftetes zentrales Umwälzrohr (11) angeordnet sind.

8. Vorrichtungen nach Anspruch 6, dadurch gekennzeichnet, dass die Anschwemm-Kerzenfilterelemente (3) ringförmig um ein zentral angeordnetes Rührelement angeordnet sind.

9. Vorrichtungen nach Anspruch 6, dadurch gekennzeichnet, dass die Anschwemm-Kerzenfilterelemente (3) oberhalb des Turbinenbelüfters angeordnet sind.

10. Vorrichtung nach den Ansprüchen 6 bis 9, dadurch gekennzeichnet, dass der Fermenterkessel (1) unterhalb der Belüftungsvorrichtung und den Anschwemm-Kerzenfilterelementen (3) eine vorzugsweise konische Verlängerung als Beruhigungszone aufweist.

## Claims

1. A method for the continuous cultivation of microorganisms in a fermenter, comprising continuously filtering the used substrate, wherein the microorganisms are filtered out by the candle filter elements (3, 3') arranged inside of the fermenter (1), and the thus deposited microorganisms are released by backwashing from the webs of the candle filterelements (3, 3').

2. A method as defined in claim 1, wherein the released and thickened microorganisms are accumulated in the lower part of the fermenter from where they are withdrawn continuously or discontinuously.

3. A method as defined in the claims 1 and 2, wherein the candle filter elements (3) are arranged in groups, and the backwashing is performed so that individual groups always carry out the filtration.

4. A method as defined in the claims 1 to 3, wherein the filtering is performed at a positiv pressure inside of the fermenter.

5. A method as defined in the claims 1 to 3, wherein a normal pressure is maintained in the fermenter and the filtering is performed by a negative pressure in a filter conduit.

6. An arrangement for carrying out the method as defined in the claim 1, wherein in a fermenter (1) a plurality of fabric coated candle filter elements (3) are arranged in groups comprising filter drain conduits and backwash conduits which are handled separately.

7. An arrangement as defined in claim 6, wherein the candle filter elements (3) are arranged annularly around an aerated central circulating tube (11).

8. An arrangement as defined in claim 6, wherein the candle filter elements (3) are arranged annularly around a central agitating element.

9. An arrangement as defined in claim 6, wherein the candle filter elements (3) are arranged above the turbine aerator.

10. An arrangement as defined in the claims 6 to 9, wherein the fermenter (1) provides under the aerating element and the candle filter elements (3) an extension which forms a stabilizing zone.

**Revendications**

1. Procédé pour la culture en continu de micro-organismes dans un fermenteur, dans lequel le substrat usé, est éliminé en continu par filtration, caractérisé par le fait que l'on sépare par filtration les micro-organismes par l'intermédiaire d'éléments filtrants (3, 3') constitués par des bougies de sédimentation, disposés à l'intérieur du fermenteur (1) et que l'on éjecte par rinçage à contre-courant, les micro-organismes déposés, des tissus de revêtement des éléments filtrants (3, 3') constitués par les bougies de sédimentation.

2. Procédé selon la revendication 1, caractérisé en ce que les micro-organismes épaissis et éjectés se rassemblent dans la partie inférieure du fermenteur d'où ils sont extraits en continu ou en discontinu.

3. Procédé selon les revendications 1 et 2, caractérisé en ce que les éléments filtrants (3) constitués par des bougies de sédimentation sont reliés par groupes et en ce que le rinçage à contre-courant est réalisé de manière à ce que des groupes déterminés assurent toujours une fonction de filtration.

4. Procédé selon les revendications 1 à 3, caractérisé en ce qu'en vue de la filtration, on opère avec une surpression dans le fermenteur.

5. Procédé selon les revendications 1 à 3, caractérisé par le fait que l'on opère dans le fermenteur à pression normale et que l'on effectue la filtration en engendrant une dépression dans le conduit de filtration.

6. Dispositif pour la mise en œuvre du procédé selon la revendication 1, caractérisé par le fait que dans un réservoir de fermentation (1) sont prévus plusieurs éléments filtrants (3) constitués par des bougies de sédimentation reliés par groupes, qui sont revêtus de tissu, qui présentent des raccords d'écoulement du filtrat et des raccords de rinçage à contre-courant disposés respectivement par groupes, et qui peuvent être mis en action individuellement.

7. Dispositif selon la revendication 6, caractérisé par le fait que les éléments filtrants (3) constitués par des bougies de sédimentation sont disposés suivant une forme annulaire autour d'un tube central de brassage (18) aéré.

8. Dispositif selon la revendication 6, caractérisé par le fait que les éléments filtrants (3) constitués par des bougies de sédimentation sont disposés suivant une forme annulaire autour d'un élément agitateur monté centralement.

9. Dispositif selon la revendication 6, caractérisé par le fait que les éléments filtrants (3) constitués par des bougies de sédimentation sont disposés au-dessus du turbo-aérateur.

10. Dispositif selon les revendications 6 à 9, caractérisé par le fait que le réservoir de fermentation (1) présente au-dessous du dispositif d'aération et des éléments filtrants (3) constitués par des bougies de sédimentation, une prolongation de préférence conique en tant que zone de modération ou stabilisation.

0 073 079

Fig.1